# EUROPÄISCHE PATENTSCHRIFT

(11) **EP 1 797 198 B1**
(45) Veröffentlichungstag und Bekanntmachung des Hinweises auf die Patenterteilung: **11.11.2009**
(21) Anmeldenummer: 05796264.9
(22) Anmeldetag: 30.09.2005
(51) Int. Cl.: C12Q 1/68

(54) **VERBESSERTES ELEKTROPHORETISCHES TRENNVERFAHREN FÜR DIE ANALYSE DER GENEXPRESSION**
IMPROVED ELECTROPHORETIC SEPARATION METHOD FOR ANALYZING GENE EXPRESSION
PROCEDE AMELIORE DE SEPARATION PAR ELECTROPHORESE POUR L'ANALYSE DE L'EXPRESSION GENIQUE

(30) Priorität: 01.10.2004 DE 102004048334
(43) Veröffentlichungstag der Anmeldung: 20.06.2007
(73) Patentinhaber: Fraunhofer-Gesellschaft zur Förderung der angewandten Forschung e.V., 80686 München (DE)
(72) Erfinder: SOHN, Kai, 73525 Schwäbisch-Gmünd (DE); RUPP, Steffen, 70569 Stuttgart (DE)
(86) Internationale Anmeldenummer: PCT/EP2005/010566
(87) Internationale Veröffentlichungsnummer: WO 2006/037563

(56) Entgegenhaltungen:
- EP-A- 0 872 559
- US-A1- 2003 017 490
- US-A1- 2003 186 296
- US-B1- 6 670 123
- SUZUKI H ET AL: "Restriction landmark cDNA scanning (RLCS): a novel cDNA display system using two-dimensional gel electrophoresis" NUCLEIC ACIDS RESEARCH, OXFORD UNIVERSITY PRESS, SURREY, GB, Bd. 24, Nr. 2, 15. Januar 1996 (1996-01-15), Seiten 289-294, XP002292437 ISSN: 0305-1048
- MAYUMI-MATSUDA K ET AL: "Scanning gene expression during neuronal cell death evoked by nerve growth factor depletion" BIOCHIMICA ET BIOPHYSICA ACTA . GENE STRUCTURE AND EXPRESSION, ELSEVIER, AMSTERDAM, NL, Bd. 1489, Nr. 2-3, 23. Dezember 1999 (1999-12-23), Seiten 293-302, XP004275539 ISSN: 0167-4781

## Beschreibung

Die vorliegende Erfindung betrifft ein verbessertes Verfahren zur quantitativen oder qualitativen Analyse der Genexpression eines biologischen Materials.

Der umfassenden funktionellen Charakterisierung von Genen kommt heute im Bereich der Biowissenschaften sowie der Medizin aber auch der Lebensmitteltechnologie eine zentrale Rolle zu. Von großer Bedeutung sind dabei sogenannte Genexpressionstudien, das heißt qualitative und quantitative Analysen zur Bestimmung der Genaktivitäten eines Organismus. Gegenwärtig werden Genexpressionsanalysen mittels der sogenannten Microarray-Technologie durchgeführt. Verfahren, die auf der Microarray-Technologie basieren, weisen jedoch gravierende Nachteile auf. Microarrays umfassen in der Regel bis zu mehrere tausend verschiedene DNA-Fragmente, die als spezifische Sonden fungieren. Die Herstellung dieser Sonden ist äußerst aufwendig und damit sehr kostenintensiv. Weiterhin wird für die Generierung dieser Sonden die Sequenzinformation der Gene, die analysiert werden sollen, vorausgesetzt. Damit beschränken sich genomweite Expressionsstudien bisher auf vollständig sequenzierte Organismen. An vielen wirtschaftlich relevanten und komplexen Organismen wie Nutzpflanzen oder Nutztieren können keine umfassenden Genexpressionsstudien durchgeführt werden, da bisher vollständige Genomsequenzen von nur wenigen ausgewählten höheren Modellorganismen wie dem Menschen oder der Maus vorliegen. Erkennbar ist daher ein wachsender Bedarf an vorteilhaften alternativen Verfahren zu der etablierten Microarray-Technologie, die die vorgenannten Nachteile nicht aufweisen.

Aus der Publikation Suzuki et al., Nucleic Acids Research, 1996,24(2):289-294, ist ein Verfahren zur Quantifizierung von markierter doppelsträngiger cDNA, welche aus der Gesamt-RNA eines Gewebes erzeugt wird, bekannt. Dazu wird die doppelsträngige cDNA markiert, einem Restriktionsverdau unterzogen, in einer ersten Dimension elektrophoretisch nach der Länge aufgetrennt und nach einem weiteren Restriktionsverdau in einer zweiten Dimension ebenfalls elektrophoretisch nach der Länge aufgetrennt. Dieses Verfahren ist auch als RLCS (restriction landmark cDNA scanning) bekannt. Entsprechende Verfahren sind aus Mayumi-Matsuda et al., Biochimica et Biophysica Acta, 1999,1489:293-302, aus der EP 0 872 559 A1 und aus der US 6,670,123 B1 bekannt. Aus der US 2003/0017490 A1 ist ein Verfahren zur Analyse von cDNA-Populationen bekannt, welche nach mehrmaligem Restriktionsverdau einer cDNA erhalten werden. Nach spezifischer Amplifikation der cDNA-Fragmente findet mindestens ein weiterer Restriktionsverdau statt, um Affinitäts-Tags von den amplifizierten Fragmenten wieder abzutrennen. Diese doppelstängigen Fragmente werden dann elektrophoretisch in der ersten Dimension nach dem Molekulargewicht und anschließend in der zweiten Dimension entlang eines denaturierenden Gradienten aufgetrennt.

Aus der US 2003/0186296 A1 ist eine zweifarbige (Mehrkanal)analyse der Genexpression von Zellen bekannt. Dazu werden einzelsträngige Nucleinsäurefragmente an einen zweidimensionalen Träger hybridisiert; doppelsträngige Komplexe werden zuvor abgetrennt, um die Hybridisierung nicht zu stören.

Der vorliegenden Erfindung liegt daher das technische Problem zugrunde, ein Verfahren zur quantitativen oder qualitativen Analyse der Genexpression eines biologischen Materials bereitzustellen, welches möglichst kostengünstige und einfach durchzuführende Genexpressionsstudien ermöglicht, insbesondere auch an komplexen biologischen Materialien, deren Sequenzinformation bisher nicht oder nur unvollständig vorliegen.

Die vorliegende Erfindung löst das ihr zugrundeliegende technische Problem durch die Bereitstellung eines Verfahrens zur quantitativen und/oder qualitativen Analyse der Genexpression eines biologischen Materials, wobei in einem ersten Verfahrensschritt a) aus dem biologischen Material RNA gewonnen, in einem zweiten Verfahrensschritt b) aus der RNA mindestens eine Population markierter doppelsträngiger cDNA erhalten, in einem dritten Verfahrensschritt c) in einem Trennsystem eine mehrdimensionale Auftrennung der mindestens einen cDNA-Population durchgeführt und in einem vierten Verfahrensschritt d) eine qualitative und/oder quantitative Auswertung der erhaltenen, mehrdimensional aufgetrennten Spots der mindestens einen cDNA-Population durchgeführt wird.

Erfindungsgemäß ist dazu vorgesehen, dass die Gesamt-RNA aus dem biologischen Material isoliert und gefällt wird, aus der Gesamt-RNA doppelsträngige cDNA hergestellt wird, die cDNA mit Restriktionsendonuklease einem Restriktionsverdau unterzogen wird, die so fragmentierte cDNA gefällt und eine Supressions-PCR zur Amplifizierung der 3-Enden der cDNA-Fragmente und gegebenenfalls ein spezifisches Amplifizieren von Subpopulationen der amplifizierten cDNA-Fragmente durchgeführt wird. Erfindungsgemäß findet ein zweidimensionales Auftrennen der erhaltenen cDNA-Population statt, wobei die cDNA-Population in der ersten Dimension nach ihrem Molekulargewicht aufgetrennt und in der zweiten Dimension nach ihrem GC-Gehalt aufgetrennt wird. Erfindungsgemäß werden die erhaltenen, mehrdimensional aufgetrennten Spots der mindestens einen cDNA-Population detektiert und qualitativ und quantitativ ausgewertet. Die Erfindung betrifft in einer bevorzugten Ausführung ein Verfahren zur quantitativen oder qualitativen Analyse der Genexpression eines biologischen Materials, wobei in einem ersten Verfahrensschritt a) aus dem biologischen Material mindestens zwei verschiedene Gesamt-RNA's oder mindestens zwei verschiedene mRNA-Populationen gewonnen, in einem zweiten Verfahrensschritt b) aus den mindestens zwei verschiedenen RNA's, insbesondere mindestens zwei verschiedenen Gesamt-RNA's oder den mindestens zwei verschiedenen mRNA-Populationen, insbesondere durch reverse Transkription, mindestens zwei verschiedene Populationen doppelsträngiger cDNA erhalten werden, diese gegebenenfalls amplifiziert werden, wobei während dieses Verfahrensschritts b) die erhaltenen mindestens zwei verschiedenen cDNA-Populationen jeweils unterschiedlich markiert werden, in einem dritten Verfahrensschritt c) in einem Trennsystem eine mehrdimensionale Aufteilung der mindestens zwei verschiedenen cDNA-Populationen durchgeführt und in einem vierten Verfahrensschritt d) eine qualitative und/oder quantitative Auswertung der erhaltenen, mehrdimensional aufgetrennten Spots der mindestens zwei cDNA-Populationen durchgeführt wird, wobei die mindestens zwei verschiedenen cDNA-Populationen gemeinsam in einem Trennsystem aufgetrennt werden.

Im Zusammenhang mit der vorliegenden Erfindung wird unter RNA sowohl mRNA als auch Gesamt-RNA verstanden. Die Erfindung sieht daher als Ausgangsmaterial sowohl die Verwendung von mRNA, insbesondere mindestens einer mRNA-Population, als auch Gesamt-RNA vor. In dem Fall, in dem mRNA oder eine mRNA-Population eingesetzt wird, stammt diese in der Regel, nach Durchführung eines Isolierungsschrittes, aus Gesamt-RNA.

Im Zusammenhang mit der vorliegenden Erfindung werden unter den Begriffen RNA, mRNA, DNA, cDNA immer, wenn nicht anders angegeben, die entsprechenden DNA- oder RNA-Moleküle verstanden.

Die Erfindung sieht daher in einem ersten Schritt vor, RNA, das heißt entweder Gesamt-RNA oder zumindest eine mRNA-Population eines biologischen Materials, bereitzustellen, aus der anschließend, insbesondere mittels reverser Transkription, mindestens eine cDNA-Population hergestellt wird, wobei diese cDNA-Population gleichzeitig mit der reversen Transkription oder anschließend markiert wirdEs ist vorgesehen, im Anschluss an die reverse Transkription, also im Anschluss an die cDNA-Synthese, eine Amplifikation der erhaltenen cDNA durchzuführen, zum Beispiel mittels PCR. Sowohl für die cDNA-Synthese als auch für die gegebenenfalls stattfindende cDNA-Amplifikation können in bevorzugter Ausführungsform Primer verwendet werden, die keine Spezifität für bestimmte Gene oder bestimmte Nucleotidsequenzen aufweisen, das heißt, Sequenz-, Gen- und/oder Organismen-unspezifisch sind. Die Primer können dabei markiert, insbesondere fluoreszenzmarkiert sein.

Mithilfe von DNA spaltenden Enzymen, zum Beispiel Restriktionsendonukleasen, vorzugsweise sequenzspezifischen Restriktionsendonukleasen, werden die Moleküle der erhaltenen cDNA-Population, das heißt die cDNA-Fragmente, geschnitten und damit vor allem das durchschnittliche Molekulargewicht der cDNA-Population festgelegt.

Im Anschluss daran wird in einem Trennsystem, insbesondere einem elektrophoretischen Trennsystem, vorzugsweise einem gelelelektrophoretischen Trennsystem, die erhaltene mindestens eine cDNA-Population in mindestens zwei verschiedenen Dimensionen, zum Beispiel zwei oder drei Dimensionen, so aufgetrennt, dass aus mindestens einer cDNA-Population ein mehrdimensionales Muster an räumlich voneinander getrennten Spots, entsteht, welches einer qualitativen und/oder quantitativen Auswertung zugeführt werden kann. Das vorliegende Verfahren ist vorteilhaft insofern, als dass unabhängig von dem Prinzip der in den herkömmlichen Microarray-Technologien eingesetzten Hybridisierung komplementärer DNA an spezifische Gensonden eine hochauflösende Auftrennung doppelsträngiger DNA ermöglicht wird. Das vorliegende Verfahren ermöglicht und dient der Analyse, auch Identifikation, von biologischen Materialien, deren Sequenzinformationen bisher nicht oder nicht vollständig vorliegen, insbesondere also Genexpressionsanalysen, ohne dass diese Analysen Kenntnisse von Sequenzdaten der zu untersuchenden biologischen Materialien oder der spezifischen Gene voraussetzt. Erfindungsgemäß werden die Nucleinsäuren der cDNA-Population mittels des erfindungsgemäß vorgesehenen, zum Beispiel zweidimensionalen, Gelsystems aufgetrennt und so in Form komplexer Spotmuster dargestellt. Durch qualitative und quantitative Auswertung können so verschiedene Spotmuster und damit verschiedene DNA-Proben beziehungsweise mRNA-Populationen miteinander verglichen und identifiziert werden, ohne dass vorab Sequenzinformationen bekannt sein müssten. Erfindungsgemäß ist es darüber hinaus auch möglich, einzelne Spots aus dem Trennsystem zu isolieren und einer Sequenzierung zu unterziehen.

Im Zusammenhang mit der vorliegenden Erfindung wird unter einem biologischen Material ein Material verstanden, das genetische Informationen enthält und sich selbst reproduzieren oder in einem biologischen System reproduziert werden kann. Beispiele für solche biologische Materialien sind Organismen, Viren, Zellen, Hefe, Bakterien, Zellsysteme, Gewebe oder dergleichen. In besonders bevorzugter Ausführungsform sieht die vorliegende Erfindung biologisches Material als Ausgangsmaterial vor, welches hinsichtlich seiner genetischen Information unbekannt ist, das heißt für die keine Sequenzinformationen des genetischen Materials vorliegt oder nur in unvollständigen Bruchstücken. In besonders bevorzugter Ausführungsform wird als biologisches Material zum Beispiel ein Nutztier angesehen, wie Ziege, Schaf, Pferd, Hund, Schwein oder dergleichen. Selbstverständlich erfasst die Erfindung auch Pflanzen insbesondere Nutzpflanzen wie Roggen, Gerste, Triticale, Mais, Weizen, Hafer, Hirse, Zuckerrohr, Futterrübe, Zuckerrübe, Reis und dergleichen.

Im Zusammenhang mit der vorliegenden Erfindung wird unter einem Spot eine nach Durchführung der Auftrennung der cDNA-Population erhaltene räumlich fokussierte lokale Ansammlung von cDNA-Molekülen verstanden, die sich im mehrdimensionalen Feld gleich verhalten haben und dementsprechend im Wesentlichen die gleiche Position im Trennsystem aufweisen.

Die Erfindung sieht in einer weiteren bevorzugten Ausführungsform vor, dass die in dem ersten Verfahrensschritt aus dem biologischen Material gewonnene RNA, das heißt Gesamt-RNA oder mindestens eine mRNA-Population, während oder nach der reversen Transkription in eine Population doppelsträngiger cDNA markiert wird, wobei die Markierung beispielsweise eine radioaktive Markierung oder eine Fluoreszenz-Markierung sein kann. Die Erfindung sieht auch eine Markierung mittels, besonders mehrerer, vor allem zwei oder drei, interkalierender Farbstoffe, zum Beispiel Cyber Green, SYBR-Green oder SYBR-Gold, vor.

Die mehrdimensionale Auftrennung mindestens einer cDNA-Population ist eine zweidimensionale Auftrennung, wobei mindestens eine cDNA-Population in einer Dimension nach ihrem Molekulargewicht aufgetrennt wird, wobei dazu in einer weiteren bevorzugten Ausführungsform eine hochauflösende DNA-Gelelektrophorese, zum Beispiel unter Einsatz von einem Polyacrylamidgel, eingesetzt wird.

Die Auftrennung der mindestens einen cDNA-Population in einer weiteren Dimension wird nach ihrem GC-Gehalt durchgeführt wird,
wobei in einer weiteren bevorzugten Ausführungsform die Auftrennung nach dem GC-Gehalt in einer denaturierenden Gradientengelelektrophorese geschieht.

In einer besonders bevorzugten Ausführungsform der vorliegenden Erfindung kann die Auftrennung nach dem GC-Gehalt auch mittels einer Temperaturgradienten-Gelelektrophorese durchgeführt werden.

In einer besonders bevorzugten Ausführungsform ist eine zweidimensionale hochauflösende Auftrennung von cDNA-Molekülen aus komplexen Populationen oder Mischungen vorgesehen, wobei die Auftrennung in einer Dimension nach dem Molekulargewicht und in einer zweiten Dimension nach dem GC-Gehalt durchgeführt wird.

Die quantitative und/oder qualitative Auswertung des nach der mehrdimensionalen Auftrennung in dem Trennsystem erhaltenen Spotmusters kann beispielsweise mittels handelsüblicher Scanner erfolgen.

In einer bevorzugten Ausführungsform der Erfindung ist vorgesehen, einzelne oder mehrere der erhaltenen Spots mittels üblicher Verfahren aus dem Trennsystem zu isolieren und anschließend einer Sequenzanalyse mittels üblicher Methoden zuzuführen.

Die Erfindung sieht in bevorzugter Ausführungsform selbstverständlich auch vor, dass mittels der erfindungsgemäßen Verfahren mehrere verschiedene Gesamt-RNA's oder mRNA-Populationen, zum Beispiel zwei verschiedene mRNA-Populationen gleichzeitig zu mehreren verschiedenen cDNA-Populationen revers transkribiert, amplifiziert und anschließend gemeinsam in einem Trennsystem einer mehrdimensionalen Auftrennung unterzogen werden. In dieser Ausführungsform ist es vorteilhaft, die verschiedenen erhaltenen cDNA-Populationen unterschiedlich zu markieren.

Das vorliegende Verfahren eignet sich insbesondere zum Einsatz in der Analytik, der Diagnostik, im Bereich der Medikamentenentwicklung (drug discovery) und der Grundlagenforschung. Darüber hinaus ist das vorliegende Verfahren vorteilhafter Weise im Bereich der Pflanzenzüchtung oder der Veterinärmedizin einsetzbar. In all den genannten Bereichen ist es wesentlich, Daten und Erkenntnisse über die Genexpression biologischer Materialien zu erhalten, das heißt Erkenntnisse über beispielsweise die Orts-, oder Zeit-, oder Entwicklungs-spezifische Aktivität von Genen des biologischen Materials, insbesondere des Organismuses zu erhalten, ohne dass dazu Sequenzinformationen über die Zielorganismen und/oder die Zielgene bekannt sind. Das erfindungsgemäße Verfahren ermöglicht eine schnelle, effiziente Analyse und Identifikation exprimierter Gene eines biologischen Materials, ohne vorab Sequenzinformationen zu diesem biologischen Material zu kennen oder einzusetzen, wobei sich je nach Auflösung des eingesetzten Trennsystems, beispielsweise eines zweidimensionalen Gels, durchaus Muster bis zu tausend verschiedenen Spots pro Trennsystem erzielen lassen, die eine eindeutige Charakterisierung und Analyse einer Genexpression ermöglichen. Das vorliegende Verfahren gewährleistet auch die Bereitstellung von Sequenzinformationen durch anschließende Sequenzanalyse.

Das erfindungsgemäße Verfahren kann in vorteilhafter Weise zur Identifizierung potentieller Virulenzfaktoren in Mikroorganismen, zum Beispiel Hefen, insbesondere Candida, vorzugsweise Candida albicans eingesetzt werden, vorzugsweise im Rahmen von GenExpressionsstudien.

Weitere vorteilhafte Ausgestaltungen ergeben sich aus den Unteransprüchen.

Die Erfindung wird anhand der folgenden Ausführungsbeispiele näher erläutert.

Die dazugehörige Figur zeigt:

Das Ergebnis einer zweidimensionalen DNA-Gelelektrophorese, nämlich ein repräsentatives Spotmuster für cDNA's aus Candida albicans.

### Beispiel 1

Wichtige Eigenschaften der Fleischqualität im Schwein definieren sich im wesentlichen über die Zusammensetzung und Strukturcharakteristika der Muskelfasern im Muskelgewebe. Die Ausprägung günstiger Eigenschaften wird dabei sehr stark von genetischen, prädisponierenden Faktoren bestimmt. Zur Identifizierung solcher Faktoren wird sowohl Muskelgewebe von einem Schwein mit hoher Fleischqualität als auch eine Gewebeprobe eines Schweins mit geringer Faserqualität genommen. Von beiden Proben wird jeweils die Gesamt-RNA isoliert und anschließend die darin enthaltene mRNA mittels reverser Transkription in Anwesenheit unterschiedlich markierter Primer bzw. Nukleotide in doppelsträngige cDNA (ds-cDNA) umgeschrieben. Diese ds-cDNA wird mit sequenzspezifischen Restriktionsendonukleasen geschnitten, um ds-DNA-Fragmente definierter Länge herzustellen. Die unterschiedlich markierten ds-cDNA-Pools werden vereinigt und mittels der mehrdimensionalen Gelelektrophorese simultan hochauflösend aufgetrennt. In der ersten Dimension werden dabei die unterschiedlichen Fragmente im Polyacrylamidgel nach ihrem jeweiligen Molekulargewicht aufgetrennt. Rechtwinklig zur Auftrennung in der ersten Dimension werden die Fragmente anschließend in der zweiten Dimension nach ihrem jeweiligen GC-Gehalt weiter separiert. Dabei wandern die DNA-Fragmente entlang eines kontinuierlichen Gradienten variierender Hybridisierungsstringenz, wobei die Formamid- und Harnstoffkonzentration im Gel kontinuierlich erhöht wird. Nach Auftrennung der markierten DNA-Fragmente in zwei Dimensionen werden die komplexen Spotmuster mittels Fluoreszenzscannern visualisiert und anschließend quantitativ und qualitativ analysiert. Spots, die ausschließlich in Proben mit hoher Fleischqualität zu detektieren sind und damit potentielle Marker darstellen, werden aus dem Gel ausgeschnitten und mittels DNA-Sequenzierung identifiziert.

### Beispiel 2

Humanpathogene Hefen haben spezifische Virulenzmechanismen entwickelt, um im entsprechenden Wirt Gewebe besiedeln zu können und damit in der Lage sind, Infektionen hervorzurufen. So verursacht zum Beispiel Candida albicans systemische Infektionen, die mit einer hohen Mortalitätsrate verbunden sind. Zur Identifizierung potentieller Virulenzfaktoren in C. albicans werden GenexpressionsStudien an klinischen Isolaten von C. albicans unter verschiedenen Bedingungen durchgeführt. Dazu wird aus C. albicans-Zellen Gesamt-RNA beziehungsweise mRNA isoliert und anschließend die entsprechende cDNA mittels reverser Transkription in an sich bekannter Weise hergestellt. Die so hergestellte cDNA wird mittels Restriktionsendonukleasen verdaut und anschließend definierte Adaptoren an die so fragmentierte cDNA ligiert. Diese adaptorligierte cDNA kann dann mittels PCR amplifiziert und mittels zweidimensionaler DNA-Gelelektrophorese aufgetrennt werden. Die so erhaltenen Spotmuster können dann qualitativ und quantitativ ausgewertet werden und die einzelnen Spots ausgestochen, mittels PCR amplifiziert und anschließend sequenziert werden. Die einzelnen experimentellen Schritte sind im folgendem detailliert aufgelistet:

### I. Anzucht von Candida albicans-Zellen und Herstellung von Zell perlen

Für die Isolierung der Gesamt-RNA wird für die Vorkultur 11 ml YPD - Medium mit Candida albicans Zellen (klinisches Isolat SC5314) angeimpft und über Nacht bei 30°C geschüttelt. Anschließend werden 50 ml YPD-Medium mit Übernachtkultur zu einer OD600 zwischen 0,15-0,20 angeimpft. Diese Kultur wird bis zu einer OD von 0,8 - 1,0 bei 30°C im Schüttler (180 Upm) wachsen gelassen. Für die Herstellung von Zellperlen wird die Zellsuspension 4 min bei 3000 xg abzentrifugiert, das Pellet im restlichen 1,0-1,5 ml Medium resuspendiert und tropfenweise in ein mit flüssigem Stickstoff befülltes 50 ml Röhrchen pipettiert. Die so eingefrorenen Proben können bei -80°C bis zur Isolation aufbewahrt werden.

### II. Isolierung der Gesamt-RNA

Für die Isolierung der Gesamt-RNA aus C. albicans sowie auch aus eukaryontischen Zellen kann ein RNeasy-Midi-Kit (Qiagen) verwendet werden. Die Zellen werden allerdings nicht mit Hilfe von Glasperlen, sondern zum Beispiel mit einer Kugelmühle der Firma Retsch aufgeschlossen.

Dabei werden die erhaltenen Zellperlen in zuvor mit flüssigem Stickstoff gekühlten Teflonbehältern mit einer Wolframkarbid-Kugel zu feinem Pulver zermahlen (shaking frequency = 30/sec, Zeit = 2 min) und anschließend in den Lyse-Puffer (Puffer RLT + 0,01 Vol% Mercaptoethanol) des Kits überführt. Zur Homogenisierung wird die Probe 1 min gevortext, dass heißt heftig geschüttelt. Anschließend werden die festen Bestandteile des Lysats zunächst 5 min bei 3500 xg abzentrifugiert und der Überstand mit 1 Volumen 70 % Ethanol versetzt und gut gemischt. Diese Lösung wird dann in 4 ml-Schritten komplett auf die RNeasy Midi-Säule gegeben und jeweils 5 min bei 3500 xg zentrifugiert. Anschließend wird die auf der Membran gebundene RNA nach Herstellerangaben zuerst mit 4 ml RW1-Puffer, dann zweimal mit jeweils 2,5 ml RPE-Puffer gewaschen. Zur Eluation der RNA wird auf die Membran 250 µl RNase-freies Wasser hinzupipettiert, die Säule 1 min bei Raumtemperatur inkubiert und anschließend 3 min bei 3500 xg zentrifugiert. Diese Schritte werden einmal wiederholt, so dass ein Endvolumen von 500 µl entsteht.

### III. Fällung der Gesamt-RNA

Für die Fällung der Gesamt-RNA werden jeweils 250 µl des Eluats in zwei 1,5 ml Reaktionsgefäße überführt und durch Zugabe von 25 µl 3 mol/l Natriumacetat, pH 5,3 und 625 µl 100% Ethanol (-20°C) über Nacht bei -20°C gefällt. Durch die 30 minütige Zentrifugation bei 4°C, 13000 xg werden die ausgefallenen Nukleinsäuren pelletiert und zur Entfernung der noch vorhandenen Salzreste anschließend zweimal mit 1 ml 70% EtOH-DEPC (-20°C) gewaschen.

Nach dem Trocknen des Gesamt - RNA - Niederschlags bei 37°C wird die RNA in 10-50 µl DEPC-H₂O aufgenommen und eine Konzentrations- und Reinheitsbestimmung mittels der UV-Spektroskopie durchgeführt.

### IV. Herstellung von ds-cDNA aus polyA+- oder Gesamt-RNA

Die Synthese von Volllängen ds-cDNA aus 0,5 - 1 µg polyA+ beziehungsweise Gesamt-RNA erfolgt nach einem modifizierten Protokoll vom SMART cDNA Library Construction Kit (BD Bioscience).

Verwendete Primer (jeweils 10 mol/l):
SMART IV Oligonucleotid:
   5'-AAGCAGTGGTATCAACGCAGAGTGGCCATTACGGCCGGG-3' **(SEQ ID Nr. 1)**
ODD_T_all:
   5'-GCGAGTCGACCGTTTTTTTTTTTTT-3' **(SEQ ID Nr. 2)**
ODD-TA:
   5'-GCGAGTCGACCGTTTTTTTTTTTTTA-3' **(SEQ ID Nr. 3)**
ODD-TG:
   5'-GCGAGTCGACCGTTTTTTTTTTTTTG-3' **(SEQ ID Nr. 4)**
ODD-TC:
   5'-GCGAGTCGACCGTTTTTTTTTTTTTC-3' **(SEQ ID Nr. 5)**
A2-Sau3a:
   5'-AAGCAGTGGTATCAACGCAGAGT-3' **(SEQ ID Nr. 6)**

Für die Erststrang cDNA Synthese werden in einem RNase-freiem Reaktionsgefäß 1-3 µl RNA [0,5 - 1,0 µg polyA+ bzw. Gesamt - RNA] mit jeweils 1 µl 10 µmol/l SMART IV Oligonucleotid- und ODD_T_all-Primer (alternativ: ein 10 µmol/l Mix aus ODD-TA, ODD-TG und ODD-TC) versetzt und bis zu einem Gesamtvolumen von 5 µl mit DEPC-H₂O aufgefüllt.

Der Ansatz wird für die Denaturierung der RNA - Sekundärstruktur zuerst 2 min bei 72°C und dann 2 min auf Eis inkubiert. Anschließend werden 2 µl 5x Erststrang Puffer (250 mmol/l Tris, pH 8,3; 30 mmol/l MgCl₂; 375 mmol/l KCl), 1 µl 20 mmol/l DTT, 1 µl 10 mmol/l dNTP Mix und 1 µl PowerScript Reverse Transkriptase hinzugegeben und die Komponenten durch vorsichtiges Auf- und Abpipettieren gemischt. Die Reverse Transkriptase Reaktion erfolgt für 1 h bei 42°C und wird durch Überführung auf Eis gestoppt. Diese Erststrang-cDNA kann nun entweder direkt in die Zweitstrangsynthese eingesetzt oder bei -20°C bis zu drei Monaten gelagert werden.

Die Zweitstrangsynthese erfolgt durch die Verwendung des BD AdvantageTM 2 Polymerase Mixes in der Long Distance (LD) - PCR. Bei dieser Mischung handelt es sich um die BD TITANIUM^{™} Taq DNA Polymerase in Kombination mit BD TaqStart^{™} Antikörper und einer geringen Menge einer proofreading Polymerase.

Im 100 µl Gesamtvolumen werden 2 µl Erststrang-cDNA, 80 µl molekularbiologisches H₂O, 10 µl 10x Advantage 2 PCR Puffer (400 mmol/l Tricine-KOH, pH8,7; 150 mmol/l KOAc; 35 mmol/l Mg(OAc)2; 37,5 µg/ml BSA; 0,05% Tween 20; 0,05% Nonidet-P40), 2 µl 50 x dNTP Mix (jeweils 10 mmol/l), 2 µl A2-Sau3A-Primer, 2 µl ODD_T_all-Primer (alternativ: ODD-TA/TC/TG -Primer-Mix) und 2 µl 50x Advantage^{™} 2 Polymerase Mix zusammen gegeben, kurz gevortext, dass heißt heftig geschüttelt, und anschließend eine "Hot-Start-PCR" mit zwei Zyklen durchgeführt.

PCR-Programm:
95°C - 0:20 min
95°C - 0:05 min
68°C - 6:00 min
95°C - 0:05 min
68°C - 6:00 min

### V. Aufreinigung der ds-cDNA

Die Aufreinigung von ds-cDNAs erfolgt mittels Säulenaufreinigung (z.B. QIAGEN QIAquick PCR Purification Kits. Die Eluation erfolgt durch Zugabe von 90 µl EB-Puffer (10mmol/l Tris-Cl, pH8,5).

### VI. Restriktionsverdau der ds-cDNA mit Restriktionsendonuklease (z.B: Rsal)

90 µl des Eluats werden im 120 µl Verdau-Ansatz mit 12 µl 10x Puffer 1 (100 mmol/l Bis Tris Propane-HCl, pH 7,0; 100 mmol/l MgCl₂; 10 mmol/l Dithiothreitol) und 12 µl 10 mg/ml BSA versetzt und nach Zugabe von 30 U Rsal 2 h bei 37°C inkubiert. Nach einer weiteren Zugabe von 15 U Rsal und Inkubation für 1 h kann die geschnittene ds-cDNA gefällt werden.

### VII. Fällung der fragmentierten ds-cDNA

Die Fällung der cDNA erfolgt durch Zugabe von 0,1 Volumen 3 mol/l Natriumacetat, pH 5,3 und 2,5 Volumen 100% EtOH direkt zum Restriktionsverdau und einer Inkubation für 2 h bei -20°C. Die Pelletierung erfolgt durch eine 25 minütige Zentrifugation (13000 xg) in der Kühlzentrifuge bei 4°C. Nach dem Waschen im 70% Ethanol [-20°C] kann das Pellet in 10 µl molekularbiologischem Wasser aufgenommen werden. Davon werden 5 µl für die Adaptorligation eingesetzt, der Rest bei -20°C eingefroren.

### VIII. Adaptersynthese

Verwendete Oligonukleotide:
Long Oligo RsaI [100µmol/l]:
   GCGTGAAGACGACAGAAAGGGCGTGGTGCGGAGGGCGGT **(SEQ ID Nr. 7)**
Short Oligo RsaI [100µmol/l]:
   ACCGCCCTCCGC **(SEQ ID Nr. 8)**

Für die Herstellung eines 20 µmol/l Adapter-Stocks (ODD_Adapter_Rsal) werden jeweils 20 µl des Long Oligo Rsal- und des Short Oligo Rsal-Primers in 100 µl Gesamtvolumen 50 mmol/l Tris-HCL, pH 7,5; 10 mmol/l MgCl₂; 10 mmol/l Dithiothreitol; 1 mmol/l ATP; 25 µg/ml BSA versetzt. Durch eine 3 min Denaturierung bei 95°C für 3 min und anschließende langsame Abkühlung (ca. 8 h) bei RT hybridisieren die komplementären Nukleotidsequenzen zu doppelsträngigen Adaptoren, die für die Ligation von Rsal-verdauter ds-cDNA eingesetzt werden können.

### IX. Adapterligation

5 µl der nach dem Rsal-Verdau gefällter ds-cDNA werden für die Adapterligation eingesetzt.

Für die Ligation werden 1 µl des 20 µmol/l Adapter-Stocklösung [ODD_Adapter_Rsal], 1 µl 10x T4-Ligase_Puffer (500 mmol/l Tris-HCL, pH 7,5; 100 mmol/l MgCl₂; 100 mmol/l Dithiothreitol; 10 mmol/l ATP; 250 µg/ml BSA), 5 µl ds-cDNA und 200 U T4-DNA-Ligase in 10 µl Gesamtvolumen zusammengemischt und über Nacht bei 16°C inkubiert.

Die Aktivität der Ligase wird am nächsten Tag durch Hitzeinaktivierung (10 min. bei 65°C) gestoppt.

Anschließend wird der 10 µl Ligationsansatz mit 90 µl Wasser auf 100 µl aufgefüllt und mittels Säulen (QIAGEN PCR Purification Kit - Protokoll) aufgereinigt.

### X. ADAPTER-PCR I (Amplifizierung der 3' Enden der Rsal verdauten ds-cDNA)

Die sogenannte Suppressions-PCR mit den Primern ODD_T_all und Primer AP1 dient vor allem zur Amplifikation der 3' Enden der cDNA.

Zur Amplifizierung der Adapter-ligierten DNA wird folgende PCR durchgeführt:
10 µl des Eluats der Adapter-ligierten ds-cDNA werden mit 2,5 µl 10x PCR-Puffer (200 mmol/l Tris-HCl, pH 8,4 ; 500 mmol/l KCl), 0,6 µl 50 mmol/l MgCl₂, 0,5 µl 5 mmol/l dNTPs, 0,25 µl 10 µmol/l AP1-Primer
(5'-TGTAGCGTGAAGACGACAGAA-3') **(SEQ ID Nr. 9),**
0,25 µl ODD_T_all-Primer
(5'-GCGAGTCGACCGTTTTTTTTTTTTT-3') **(SEQ ID Nr. 2),**
10,65 µl molekularbiologisches Wasser sowie 2 U Taq-Polymerase vermischt und anschließend mit folgendem PCR-Programm amplifiziert:
PCR-Programm:
   1. 95°C - 0:05 min
   2. 65°C - 0:30 min
   3. 72°C - 1:00 min

Schritte 2 und 3 werden 19 mal wiederholt (Gesamtzyklenzahl: 20).

Die so amplifizierte cDNA kann anschließend in einer zweiten PCR (Adapter-PCR II) spezifisch amplifiziert werden, wobei sogenannte anchored Primer Verwendung finden
Primer 1:
   AGGGCGTGGTGCGGAGGGCGGTCCNN **(SEQ ID Nr. 10),**
   wobei N die Nucleotide A, G, C oder T repräsentieren kann und damit 16 Primer 1-Varianten möglich sind; sowie
Primer 2:
   5'-GCGAGTCGACCGTTTTTTTTTTTTTVN-3' **(SEQ ID Nr. 11),**
   wobei V die Nucleotide A, G, oder C und N die Nucleotide A, G, C oder T repräsentieren können und somit 12 Primer 2-Varianten möglich sind).

### XI. ADAPTER-PCR II

Für die spezifische Amplifizierung von Subpopulationen von amplifizierten cDNA-Fragmenten aus der Adapter-PCRI wird folgender Ansatz gewählt:
Die amplifizierten cDNA-Fragmente aus der Adapter-PCR I werden 1:30 mit Wasser verdünnt. 10 µl dieser Verdünnung werden mit 10 µl 10x PCR-Puffer (200 mmol/l Tris-HCl, pH 8,4 ; 500 mmol/l KCl), 3 µl 50 mmol/l MgCl₂, 4 µl 5 mmol/l dNTPs, 2,5 µl 10 µmol/l Primer 1
   (5'- GGGCGTGGTGCGGAGGGCGGTCCNN-3') **(SEQ ID Nr. 10),**
2,5 µl 10 µmol/l Primer 2
   (5'- GCGAGTCGACCGTTTTTTTTTTTTTVN -3') **(SEQ ID Nr. 11),**
65,5 µl molekularbiologisches Wasser sowie 20 U Taq-Polymerase vermischt und anschließend mit folgendem PCR-Programm amplifiziert:
PCR-Programm:
   1. 95°C - 0:05 min
   2. 65°C - 0:30 min
   3. 72°C - 1:00 min
Schritte 2 und 3 werden 20 mal wiederholt (Gesamtzyklenzahl: 21).

### XII. ZWEIDIMENSIONALE AUFTRENNUNG DER cDNA

Die amplifizierten cDNA-Fragmente aus der Adapter-PCR II werden anschließend mittels zweidimensionaler Gelelektrophorese hochauflösend aufgetrennt. Dazu werden die Proben zunächst in der ersten Dimension in einem nicht-denaturierenden Polyacrylamidgel aufgetragen. Zusammensetzung des Gels für die erste Dimension (Auftrennung nach Molekulargewicht): 8% Acrylamid (Verhältnis Acrylamid : Bisacrylamid 37,5 : 1) in 1xTAE (40 mmol/l Tris; 20 mmol/l Eisessig; 1 mmol/l EDTA, pH 8,0). Die Proben werden zwischen 2-4 Stunden bei konstanter Spannung bei 200 Volt elektrophoretisiert. Anschließend wird die Spur der ersten Dimension ausgeschnitten und zur Auftrennung in der zweiten Dimension auf ein Polyacrylamidgel, das einen Konzentrationsgradient an Harnstoff und Formamid enthält, aufgelegt. Das Polyacrylamidgel der zweiten Dimension kann dabei entweder eine konstante Konzentration oder ebenfalls einen Konzentrationsgradienten an Acrylamid aufweisen. Zusammensetzung des Gels für die zweite Dimension: 8 % Acrylamid (Verhältnis Acrylamid : Bisacrylamid 37,5 : 1) in 1xTAE (40 mmol/l Tris; 20 mmol/l Eisessig; 1 mmol/l EDTA, pH 8,0); kontinuierlicher Gradient von 0,7 mol/l Harnstoff, 4 % Formamid oben, bis 2,8 mol/l Harnstoff, 16 % Formamid unten. Die Fragmente werden in dieser zweiten Dimension bei konstanter Temperatur bei 60°C für 15 Stunden bei konstanter Spannung bei 100 Volt elektrophoretisiert.

### XIII. DETEKTION DER DNA-FRAGMENTE

Das 2D-Gel wird zur Visualisierung der DNA-Spots mittels SYBR-Gold gefärbt. Bei Verwendung fluoreszenzmarkierter Primer 1 und Primer 2 in der Adapter-PCR II entfällt eine Färbung des 2D-Gels mittels SYBR-Gold. Die Detektion der DNA-Spots erfolgt mittels handelsüblicher Fluoreszenzdokumentationssystemen.

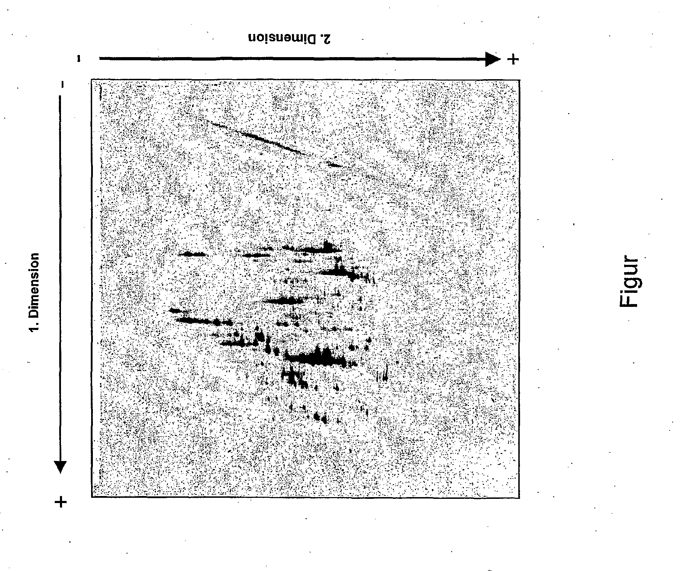

Die Figur zeigt ein repräsentatives Spotmuster für cDNAs aus C. albicans nach zweidimensionaler Auftrennung und Färbung mittels SYBR-Gold.
1. Dimension: 8% TAE-Polyacrylamidgel (Auftrennung nach Molekulargewicht)
2. Dimension: Denaturierende Gradientengelelektrophorese 8% Acrylamid, kontinuierlicher Denaturierungsgradient von 4% Formamid + 0,7 mol/l Harnstoff bis 16% Formamid + 2,8 mol/l Harnstoff (Auftrennung nach GC-Gehalt)

### SEQUENCE LISTING

<110> Fraunhofer-Gesellschaft zur Förderung der angewandten Forschung e.V.
<120> Verbessertes elektrophoretisches Trennverfahren für die Analyse der Genexpression
<130> 26293 WO
<150> DE102004048334.5-41
   <151> 2004-10-04
<160> 11
<170> PatentIn version 3.3
<210> 1
   <211> 39
   <212> DNA
   <213> Artificial
<220>
   <223> Candida albicans
<400> 1
   aagcagtggt atcaacgcag agtggccatt acggccggg
   39
<210> 2
   <211> 25
   <212> DNA
   <213> Artificial
<220>
   <223> Candida albicans
<400> 2
   gcgagtcgac cgtttttttt ttttt 25
<210> 3
   <211> 26
   <212> DNA
   <213> Artificial
<220>
   <223> Candida albicans
<400> 3
   gcgagtcgac cgtttttttt ttttta 26
<210> 4
   <211> 26
   <212> DNA
   <213> Artificial
<220>
   <223> Candida albicans
<400> 4
   gcgagtcgac cgtttttttt tttttg 26
<210> 5
   <211> 26
   <212> DNA
   <213> Artificial
<220>
   <223> Candida albicans
<400> 5
   gcgagtcgac cgtttttttt tttttc 26
<210> 6
   <211> 23
   <212> DNA
   <213> Artificial
<220>
   <223> Candida albicans
<400> 6
   aagcagtggt atcaacgcag agt 23
<210> 7
   <211> 39
   <212> DNA
   <213> Artificial
<220>
   <223> Candida Albicans
<400> 7
   gcgtgaagac gacagaaagg gcgtggtgcg gagggcggt 39
<210> 8
   <211> 12
   <212> DNA
   <213> Artificial
<220>
   <223> Candida albicans
<400> 8
   accgccctcc gc 12
<210> 9
   <211> 21
   <212> DNA
   <213> Artificial
<220>
   <223> Candida albicans
<400> 9
   tgtagcgtga agacgacaga a 21
<210> 10
   <211> 26
   <212> DNA
   <213> Artificial
<220>
   <223> Candida albicans
<220>
   <221> misc_feature
   <222> (25)..(26)
   <223> n is a, c, g, or t
<400> 10
   agggcgtggt gcggagggcg gtccnn
   26
<210> 11
   <211> 27
   <212> DNA
   <213> Artificial
<220>
   <223> Candida albicans, v is a, g or c
<220>
   <221> misc_feature
   <222> (27)..(27)
   <223> n is a, c, g, or t
<400> 11
   gcgagtcgac cgtttttttt tttttvn
   27

## Patentansprüche

1. Verfahren zur quantitativen oder qualitativen Analyse der Genexpression eines biologischen Materials, enthaltend die Schritte:
Isolieren und Fällen der Gesamt-RNA aus dem biologischen Material;
Herstellen von doppelsträngiger cDNA aus der Gesamt-RNA;
Restriktionsverdauen der cDNA mit Restriktionsendonuclease;
Fällen der fragmentierten cDNA;
Durchführen einer Suppressions-PCR zur Amplifizierung der 3'-Enden der cDNA-Fragmente und gegebenenfalls spezifisches Amplifizieren von Subpopulationen der amplifizierten cDNA-Fragmente;
zweidimensionales Auftrennen der erhaltenen cDNA-Population, wobei die cDNA-Population in der ersten Dimension nach ihrem Molekulargewicht und in der zweiten Dimension nach ihrem GC-Gehalt aufgetrennt wird; und
Detektieren und qualitatives und/oder quantitatives Auswerten der erhaltenen, mehrdimensional aufgetrennten Spots der mindestens einen cDNA-Population.

2. Verfahren nach Anspruch 1, wobei die Auftrennung nach dem Molekulargewicht mittels DNA-Gelelektrophoresesystem erfolgt.

3. Verfahren nach Anspruch 1 oder 2, wobei die Auftrennung nach dem GC-Gehalt in einer denaturierenden Gradientengelelektrophorese erfolgt.

4. Verfahren nach einem der vorhergehenden Ansprüche, wobei in einem weiteren Schritt eine Sequenzierung von DNA-Molekülen aus mindestens einem der in dem Trennsystem erhaltenen und daraus isolierten cDNA-Spot erfolgt.

5. Verfahren nach einem der vorhergehenden Ansprüche, wobei die doppelsträngige cDNA-Population mit interkalierenden Farbstoffen oder radioaktiv- oder fluoreszenz-markiert ist.

6. Verfahren nach einem der vorhergehenden Ansprüche, wobei
aus dem biologischen Material mindestens zwei verschiedene Gesamt-RNA's oder mindestens zwei verschiedene mRNA-Populationen gewonnen;
aus den mindestens zwei verschiedenen RNA's, insbesondere mindestens zwei verschiedenen Gesamt-RNA's oder den mindestens zwei verschiedenen mRNA-Populationen, insbesondere durch reverse Transkription, mindestens zwei verschiedene Populationen doppelsträngiger cDNA erhalten werden, wobei während dieses Verfahrensschritts die mindestens zwei verschiedenen Populationen doppelsträngiger cDNA's jeweils unterschiedlich markiert werden;
in einem Trennsystem eine gemeinsame mehrdimensionale Aufteilung der mindestens zwei verschiedenen cDNA-Populationen durchgeführt wird; und
eine qualitative und/oder quantitative Auswertung der erhaltenen, mehrdimensional aufgetrennten Spots der mindestens zwei cDNA-Populationen durchgeführt wird.

## Claims

1. Method for quantitative or qualitative analysis of the gene expression of a biological material, containing the steps:
isolation and precipitation of the total RNA from the biological material;
production of double-stranded cDNA from the total RNA;
restriction digestion of the cDNA with restriction endonuclease;
precipitation of the fragmented cDNA;
implementation of a suppression PCR in order to amplify the 3' ends of the cDNA fragments and if necessary specific amplification of sub-populations of the amplified cDNA fragments;
two-dimensional separation of the obtained cDNA population, the cDNA population being separated in the first dimension according to the molecular weight thereof and in the second dimension according to the GC content thereof; and
detection and qualitative and/or quantitative evaluation of the obtained, multi-dimensionally separated spots of the at least one cDNA population.

2. Method according to claim 1, the separation according to the molecular weight being effected by means of a DNA gel electrophoresis system.

3. Method according to claim 1 or 2, the separation according to the GC content being effected in a denaturing gradient gel electrophoresis.

4. Method according to one of the preceding claims, a sequencing of DNA molecules from at least one of the cDNA spots which are obtained in the separation system and isolated therefrom being effected in a further step.

5. Method according to one of the preceding claims, the double-stranded cDNA population being marked with intercalating colourants or being radioactively- or fluorescence-marked.

6. Method according to one of the preceding claims,
at least two different total RNAs or at least two different mRNA populations being obtained from the biological material;
at least two different populations of double-stranded cDNA being obtained from the at least two different RNAs, in particular from at least two different total RNAs or the at least two different mRNA populations, in particular by reverse transcription, the at least two different populations of double-stranded cDNAs respectively being marked differently during this method step;
a common multi-dimensional separation of the at least two different cDNA populations being implemented in a separation system; and
a qualitative and/or quantitative evaluation of the obtained multi-dimensionally separated spots of the at least two cDNA populations being implemented.

## Revendications

1. Procédé d'analyse quantitative ou qualitative de l'expression génique d'un matériau biologique comportant les étapes consistant à :
- isoler et précipiter l'ARN total du matériau biologique,
- préparer de l' ADNc et à double brin à partir de l'ARN total,
- fragmenter l'ADNc par restriction avec une endonucléase de restriction,
- précipiter l'ADNc fragmenté,
- effectuer une amplification par PCR pour amplifier les extrémités 3' des fragments d'ADNc et le cas échéant amplification spécifique de sous populations des fragments d'ADNc amplifiés,
- effectuer une séparation bidimensionnelle de la population d'ADNc obtenue, la population d'ADNc étant séparée dans la première dimension selon son poids moléculaire et dans la seconde dimension selon sa teneur en GC, et
- détecter et effectuer une évaluation qualitative et/ ou quantitative des images multidimensionnelles obtenues par séparation d'au moins une population d'ADNc.

2. Procédé conforme à la revendication 1, selon lequel
la séparation selon le poids moléculaire s'effectue au moyen d'un système d'électrophorèse sur gel d'ADN.

3. Procédé conforme à la revendication 1 ou 2, selon lequel
la séparation selon la teneur en GC est effectuée par électrophorèse sur gel en gradient dénaturant.

4. Procédé conforme à l'une des revendications précédentes, selon lequel
dans une autre étape, on effectue un séquençage de molécules d'ADN à partir d'au moins l'une des images d'ADNc obtenues dans le système de séparation et isolées de ce système.

5. Procédé conforme à l'une des revendications précédentes, selon lequel
la population d'ADNc à double brin est marquée par des colorants d'ADN, ou par radioactivité ou fluorescence.

6. Procédé conforme à l'une des revendications précédentes, selon lequel
à partir du matériau biologique, on obtient au moins deux ARN totaux différents ou au moins deux populations d'ARNm différentes,
à partir des au moins deux ARN différents, en particulier des au moins deux ARN totaux différents, ou des au moins deux populations d'ARNm différentes, on obtient, en particulier par transcription inverse au moins deux populations différentes d'ADNc à double brin, au cours de cette étape de procédé, les au moins deux populations différentes d'ADNc à double brin étant respectivement marquées différemment,
on effectue une répartition multidimensionnelle commune des au moins deux populations d'ADNc différentes dans un système de séparation, et
on effectue une évaluation qualitative et/ou quantitative des images ayant subi un séparation multidimensionnelle obtenues des au moins deux populations d'ADNc.
